# EUROPEAN PATENT APPLICATION

(11) **EP 4 187 245 A1**
(43) Date of publication of application: **31.05.2023**
(21) Application number: 21306653.3
(22) Date of filing: 29.11.2021
(51) Int. Cl.: G01N 33/50, G01N 33/574

(54) **METHODS FOR ASSESSING THE TOXICITY AND EFFICACITY OF RADIATION THERAPY**

(71) Applicant: Institut Curie, 75248 Paris Cedex 05 (FR); INSERM (Institut National de la Santé et de la Recherche Médicale), 75013 Paris (FR); Centre national de la recherche scientifique, 75016 Paris (FR); Université Paris-Saclay, 91190 Gif-sur-Yvette (FR)
(72) Inventor: The designation of the inventor has not yet been filed
(74) Representative: Cabinet Becker et Associés

(57) **Abstract**

The invention relates to the field of radiotherapy and cancer prognosis, diagnosis and therapeutics. More particularly, the invention relates to methods for assessing radiation-induced toxicity and to methods for identifying and selecting subjects for treatment with radiation therapy. Also provided herein are methods for treating subjects who respond to radiation therapy and for determining the efficacy of such radiation therapy.

## Description

### FIELD OF THE INVENTION

The invention relates to the field of radiotherapy and cancer prognosis, diagnosis and therapeutics. More particularly, the invention relates to methods for assessing radiation-induced toxicity and to methods for identifying and selecting subjects for treatment with radiation therapy. Also provided herein are methods for treating subjects who respond to radiation therapy and for determining the efficacy of such radiation therapy.

### BACKGROUND OF THE INVENTION

Radiation therapy or radiotherapy (RT) is the most widely used and effective anti-tumor therapy. However, it is technically impossible to concentrate the impact of ionizing radiation exclusively on tumors, thus normal tissues, which are included into the treatment plan, are unavoidably exposed (Vinnikov V, Hande MP , Wilkins R, Wojcik A , Zubizarreta E, Belyakov O; Prediction of the Acute or Late Radiation Toxicity Effects in Radiotherapy Subjects Using Ex Vivo Induced Biodosimetric Markers: A Review; J. Pers. Medecine 2020, 10, 285). RT is not specific to cancer cells, and radiation-induced cytotoxic effects occur both in the tumor and in normal tissues.

Thus, preventing radiation-induced healthy tissue injury has always been a topic of particular interest in radiotherapy research. In radiation therapy, there is a wide variation in the reaction of normal tissues, and in many situations, the severity of these reactions limits the dose of radiation therapy that can be administered to the tumor (Baumann, M.; Krause, M.; Overgaard, J.; Debus, J.; Bentzen, S.M.; Daartz, J.; Richter, C.; Zips, D.; Bortfeld, T. Radiation oncology in the era of precision medicine. Nat. Rev. Cancer. 2016, 16, 234-249). Well documented clinical experience shows that comprehensive normal tissue reactions occur due to differences in individual normal tissue sensitivity. If such variation in normal tissue reactions is due to differences in intrinsic cellular radiosensitivity, it should be possible to predict the former based on the measurement of the latter (Burnet, N.G.; Nyman, J.; Turesson, I.; Wurm, R.; Yarnold, J.R.; Peacock, J.H. The relationship between cellular radiation sensitivity and tissue response may provide the basis for individualising radiotherapy schedules. Radiother. Oncol. 1994, 33, 228-238). Ideally, results of such testing would provide a basis for personalized treatment, i.e., individualizing RT schemes (Tucker, S.L.; Geara, F.B.; Peters, L.J.; Brock, W.A. How much could the radiotherapy dose be altered for individual subjects based on a predictive assay of normal-tissue radiosensitivity? Radiother. Oncol. 1996, 38, 103-113).

Many studies were carried out in order to establish whether normal cell radiosensitivity correlates with the grade of normal tissue toxicity (NTT) in RT subjects (Granzotto, A.; Joubert, A.; Viau, M.; Devic, C.; Maalouf, M.; Thomas, C.; Vogin, G.; Malek, K.; Colin, C.; Balosso, J.; et al. Individual response to ionising radiation: What predictive assay(s) to choose? C. R. Biol. 2011, 334, 140-157). Progress in this research, however, has been hampered by the difficulty in the translation of adverse reactions from the clinic to the laboratory.

Furthermore, whereas techniques of assessing toxic effects of radiotherapy on tumors and derived organoids are well documented, standard methods using healthy tissues to monitor toxicity of irradiation are not. For example, radiation-induced damage to the lung leads to an acute pneumonitis followed by abortive regenerative processes that, together with chronic inflammation, foster fibrosis development in the months following radiation (Fouillade et al., FLASH Irradiation Spares Lung Progenitor Cells and Limits the Incidence of Radio-induced Senescence Clin.Cancer Research, December 3, 2019; DOI: 10.1158/1078-0432.CCR-19-1440.; Yarnold J, Brotons MC. Pathogenetic mechanisms in radiation fibrosis. Radiother Oncol 2010;97:149-61). The actual solution to evaluate lung toxicity therefore relies on the analysis of pulmonary fibrosis development by CT-scan and histological analysis. These solutions are time-consuming (i.e., several months needed before getting final results) and pose some ethical issues (i.e., large cohorts of animals), thus preventing the screening of a large number of conditions.

Thus, there is still a need for new efficient prognostic and diagnostic tools in the field of radiotherapy, especially for new assays for estimating radiation toxicity, that would be useful in preclinical studies and cancer therapy.

### SUMMARY OF THE INVENTION

The purpose of the present invention is to provide novel methods for assessing the toxicity of radiation therapy comprising the use of organotypic slice comprising healthy tissue samples from subjects and measuring cell proliferation after irradiation, thereby determining the radiation-induced toxicity. Optionally, these methods could be supplemented by methods for predicting and/or assessing the responsiveness of subjects to radiotherapy or the efficacy of radiotherapy in subjects having cancer.

A particular object of the present invention relates to an *ex vivo* method for assessing radiation-induced toxicity, wherein the method comprises:
- irradiating an organotypic slice comprising healthy tissue;
- measuring cell proliferation in the irradiated organotypic slice; and
- determining the radiation-induced toxicity based on the measured cell proliferation in the irradiated organotypic slice.

Another particular object of this invention relates to the use of an organotypic slice comprising healthy tissue for assessing radiation-induced toxicity by measuring cell proliferation in the organotypic slice after irradiation of said organotypic slice.

Preferably, the organotypic slice according to the invention is prepared from a biopsy or tissue resection from a subject, for example from lung, brain, gut or skin. More preferably, the organotypic slice is a lung organotypic slice prepared from a lobectomy of a subject having a lung cancer.

Preferably, the step of irradiating an organotypic slice according to the invention is performed using FLASH radiotherapy (FLASH-RT), FLASH proton irradiation, proton radiotherapy, proton minibeam radiation therapy, gamma rays, brachytherapy, unsealed source radiotherapy, tomotherapy or external beam radiotherapy selected from superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT) or 3-dimensional conformal radiation therapy (3D-CRT).

Preferably, the step of measuring cell proliferation according to the invention is performed by counting a number of dividing cells in the organotypic slice, for example by using an assay based on incorporation of a 5-ethynyl-2'-deoxyuridine (EdU).

A further object of this invention relates to a method for selecting a subject as adapted for radiotherapy, the method comprising assessing radiation-induced toxicity of said subject by performing the method for assessing radiation-induced toxicity according to the present invention on an organotypic slice from said subject, and selecting the subject as adapted for radiotherapy if the radiation-induced toxicity of said subject is low and/or selecting the subject being not adapted for radiotherapy if the radiation-induced toxicity of said subject is high.

A further object of this invention relates to a method for providing information for adapting radiotherapy in a subject based on radiation-induced toxicity of said subject, the method comprising assessing the radiation-induced toxicity of said subject by performing the method for assessing radiation-induced toxicity according to the present invention on an organotypic slice from said subject, and defining a personalized or optimized radiation therapy regimen for the subject based on radiation-induced toxicity of said subject.

A further object of this invention relates to a method for treating a subject having a cancer by radiotherapy, the method comprising assessing the radiation-induced toxicity of said subject by performing the method for assessing radiation-induced toxicity according to the present invention on an organotypic slice from said subject, and treating said subject by radiation therapy, optionally with a personalized or optimized radiation therapy regimen based on radiation-induced toxicity of said subject, preferably the subject having a cancer relapse.

The invention may be used in any mammalian subject, particularly any human subject, to predict the sensitivity, toxicity and/or responsiveness of a subject to radiotherapy and to diagnose, prognose and treat cancer in the subject.

### BRIEF DESCRIPTION OF THE FIGURES

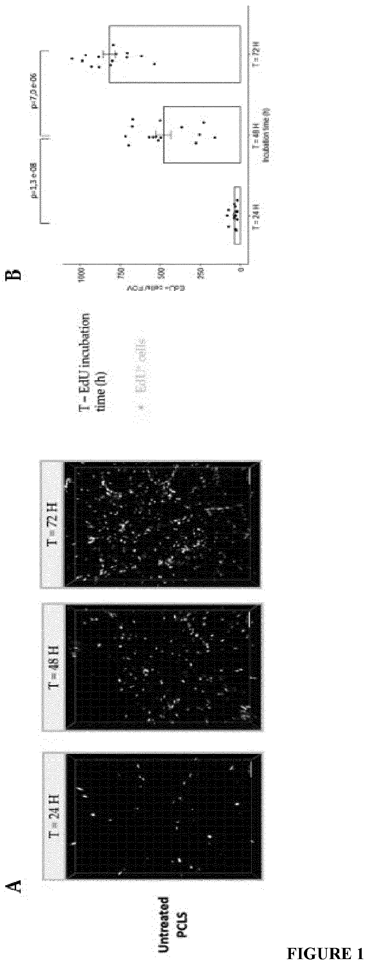

**Figure 1****:** Estimation of proliferating cells within PCLS. (A) Representative 3D reconstruction images analyzed by IMARIS software showing the EdU+ cells after 24 h, 48 h or 72 h incubation in EdU for non-irradiated PCLS. Images were obtained by confocal microscopy on 50 stacks of 3 µm with a 10X objective. Scale bar of 100 µm. (B) Quantive analysis of the number of EdU+/FOV shows a strong increase in the number of EdU+ cells over time (min 4 FOV for n=3 PCLS).

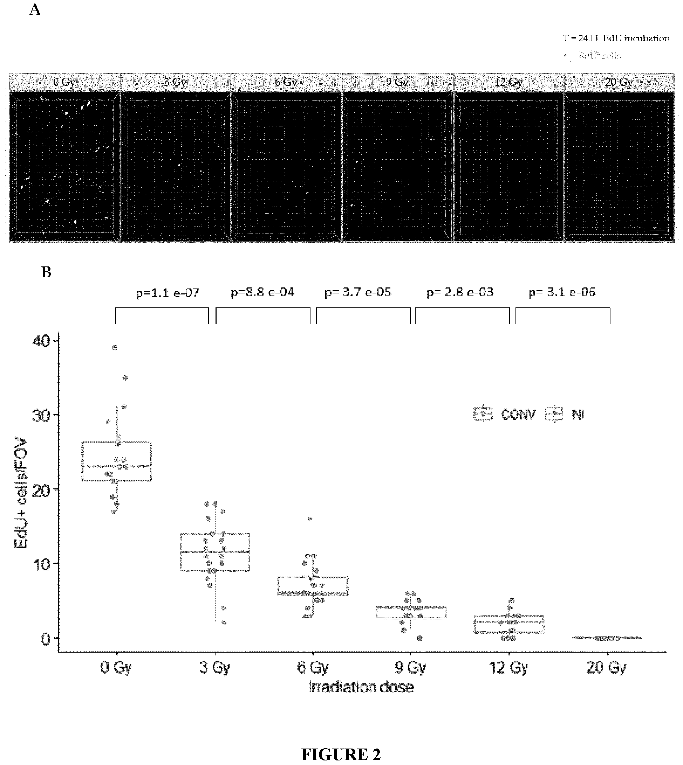

**Figure 2****:** Evaluation of cell division capacity after radiation within PCLS (Name of the test). (A) Representative 3D reconstruction obtained on IMARIS software showing the EdU⁺ cells per FOV at 24 h after exposure to increased doses of radiation. Images were acquired on a confocal microscopy with a 10X objective. Scale bar represents 100 µm. (B) Image analysis and quantification shows a significant decrease in the number of EdU⁺ cells 24 h after radiation exposure. Quantification for each dose is performed on five FOV per PCLS (n = 4).

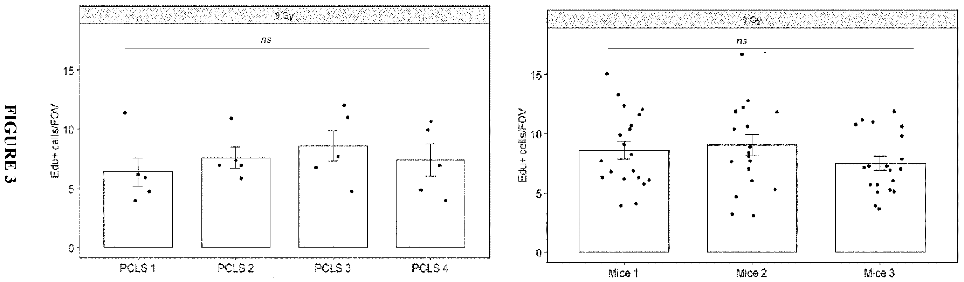

**Figure 3****:** Determination of the reproducibility of the method of assessing the toxicity of radiation therapy according to the invention. (A) Variability of the method analysis between PCLS obtained from the same mouse was not significant 24 hours after irradiation with a dose of 9 Gy. (B) Quantification of the number of EdU⁺ cells from PCLS obtained from different mice and irradiated at 9 Gy did not reveal any significant differences between the different mice analyzed.

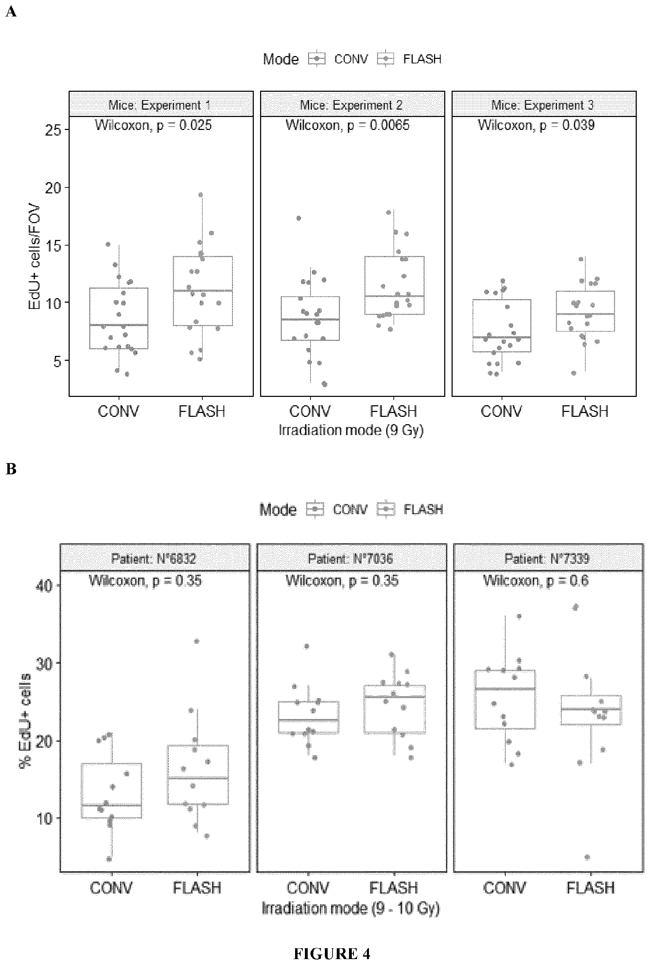

**Figure 4****:** Estimation of the sensitivity of the method of assessing the toxicity of radiation therapy according to the invention. (A) Quantification of the number of EdU⁺ cells in PCLS with [name of the test] shows a significant increase in EdU⁺ cells after FLASH irradiation compared to CONV, demonstrating the sensitivity of [name of the test]. (B) However, the analysis of EdU⁺ cells in LBSC obtained from subjects, another lung *in vitro* model, was not able to detect a difference in the number of proliferating cells after CONV vs. FLASH irradiation. Non-parametric Wilcoxon test was performed on the raw data to evaluate difference significance.

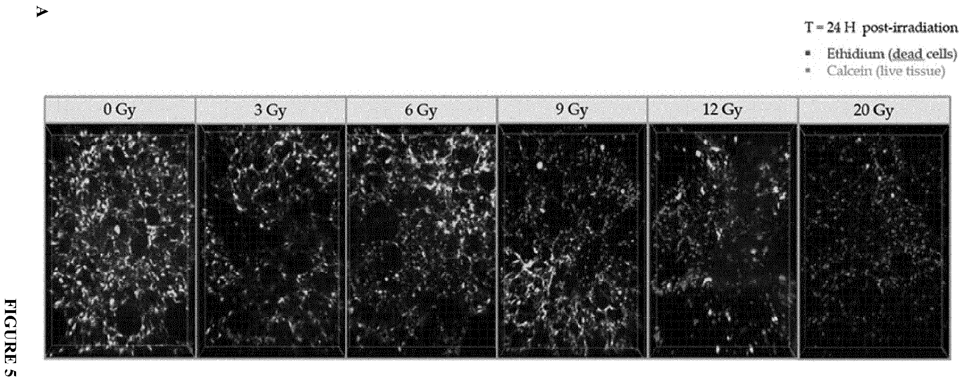

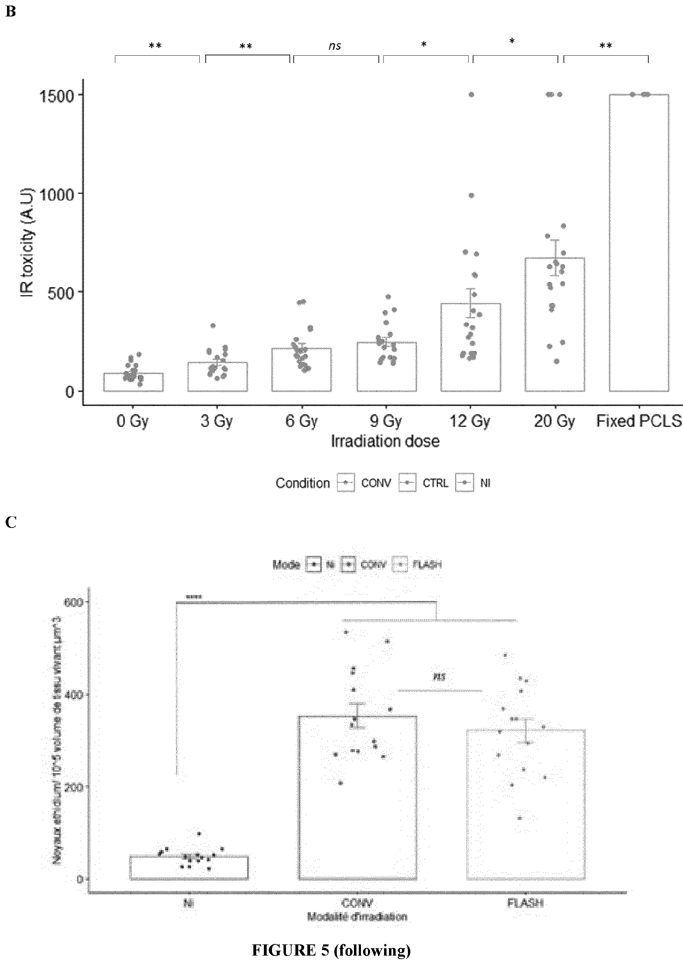

**Figure 5****:** Analysis of viability in organotypic lung slices. (A) Representative 3D reconstruction obtained on IMARIS software representing the Ethidium positive dead cell nuclei (in red) compared to the volume of living tissue labeled by Calcein (in green) after acquisition of our observation fields at 24 h post-irradiation for different doses [0; 3; 6; 9; 12 and 20 Gy]. The thresholding of our labeling was determined on the non-irradiated condition. (B) Quantification showing a dose dependent increase in the ratio of dead cells to live tissue volume at 24 h post-irradiation. Images were obtained by confocal microscopy on 20 stacks of 3 µm (60 µm total) at x20 objective. Scale bar of 50 µm. (C) Quantification of viability at 24 h post-irradiation showing no difference between conventional and FLASH irradiation modalities. Quantification for each irradiation dose is performed on n=5 observation fields for n=4 PCLS. ns p>0,05 ; ^{∗}p<0,05 ; ^{∗∗}p<0,01.

### DETAILED DESCRIPTION OF THE INVENTION

The present invention relates to new methods for assessing toxic effects associated with radiation therapy. More specifically, the methods of the invention allow to determine the radiation-induced toxicity by using organotypic slices and by measuring cell proliferation in said organotypic slices post irradiation. The invention is thus based on a measure of cell proliferation in the irradiated organotypic slices, as an indication of radiation-induced toxicity.

As shown in the experimental part, the inventors have developed an assay for evaluating the impact of the radiation therapy on healthy tissues and for selecting subjects responsive to radiation therapy. Such an assay may be used in preclinical studies but also in cancer prognosis, diagnosis and treatment by radiation therapy.

The assay designed by the inventors has to be reproductible and sensitive. As shown in the examples, this assay meets these requirements and, among numerous possibilities tested by the inventors, this assay couldn't be developed solely by routine experiments. Indeed, at the inventors' knowledge, lung organotypic slices have never been previously used for studying effect of radiotherapy.

Within the context of the present invention, the term **"radiation therapy"** or **"radiotherapy"** includes any type of radiation therapy known in the art. Suitable examples of radiation therapies include, but are not limited to external beam radiotherapy (such as superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT), 3-dimensional conformal radiation therapy (3D-CRT) and the like); brachytherapy; unsealed source radiotherapy; tomotherapy; and the like. Gamma rays are another form of photons used in radiotherapy. Gamma rays are produced spontaneously as certain elements (such as radium, uranium, and cobalt 60) release radiation as they decompose, or decay. In some embodiments, radiotherapy may be proton radiotherapy or proton minibeam radiation therapy. Proton radiotherapy is an ultra-precise form of radiotherapy that uses proton beams (Prezado Y, Jouvion G, Guardiola C, Gonzalez W, Juchaux M, Bergs J, Nauraye C, Labiod D, De Marzi L, Pouzoulet F, Patriarca A, Dendale R. Tumor Control in RG2 Glioma-Bearing Rats: A Comparison Between Proton Minibeam Therapy and Standard Proton Therapy. Int J Radiat Oncol Biol Phys. 2019 Jun 1;104(2):266-271. doi: 10.1016/j .ijrobp.2019.01.080; Prezado Y, Jouvion G, Patriarca A, Nauraye C, Guardiola C, Juchaux M, Lamirault C, Labiod D, Jourdain L, Sebrie C, Dendale R, Gonzalez W, Pouzoulet F. Proton minibeam radiation therapy widens the therapeutic index for high-grade gliomas. Sci Rep. 2018 Nov 7;8(1): 16479. doi: 10.1038/s41598-018-34796-8). Radiotherapy may also be FLASH radiotherapy (FLASH-RT) or FLASH proton irradiation. FLASH radiotherapy involves the ultra-fast delivery of radiation treatment at dose rates several orders of magnitude greater than those currently in routine clinical practice (ultra-high dose rate) (Favaudon V, Fouillade C, Vozenin MC. The radiotherapy FLASH to save healthy tissues. Med Sci (Paris) 2015; 31 : 121-123. DOI: 10.1051/medsci/20153102002); Patriarca A., Fouillade C. M., Martin F., Pouzoulet F., Nauraye C., et al. Experimental set-up for FLASH proton irradiation of small animals using a clinical system. Int J Radiat Oncol Biol Phys, 102 (2018), pp. 619-626. doi: 10.1016/j.ijrobp.2018.06.403. Epub 2018 Jul 11).

In a first embodiment, the present invention provides an *ex vivo* method for assessing radiation-induced toxicity, wherein the method comprises:
- irradiating an organotypic slice comprising healthy tissue;
- measuring cell proliferation in the irradiated organotypic slice; and
- determining the radiation-induced toxicity based on the measured cell proliferation in the irradiated organotypic slice.

In another embodiment, the present invention relates to the use of an organotypic slice, in particular an organotypic slice comprising healthy tissue, for assessing radiation-induced toxicity by measuring cell proliferation in the organotypic slice after irradiation of said organotypic slice.

### Organotypic slice irradiation

Within the context of the present invention, the term "organotypic slice" designates a fragment of tissue prepared from any organ of a mammalian subject. The organotypic slice according to the invention may comprise healthy and/or cancer tissue. Preferably, the organotypic slice according to the invention comprises healthy tissue. In particular, the organotypic slice may only comprise healthy tissue and no cancer tissue. In another embodiment, the organotypic slice according to the invention comprises both healthy and cancer tissues.

The term "organotypic slice" according to the invention also includes any organotypic slice culture corresponding to an organotypic slice which is cultured in a suitable culture medium. It refers to sections or explants of tissue which are maintained in culture (Kang (2016) Sci. Rep. 6:28798 | DOI: 10.1038/srep28798; Seil (1979) Review in Neuroscience 4: 105-177; Gahwiler (1981) J Neurosci Meth 4:329-342; Gahwiler (1984) Neuroscience 11:751- 760, Gahwiler (1988) Trends Neurosci 11:484-490; Stoppini et al. (1991) J Neurosci Methods 37: 173-182). A skilled artisan can readily employ art known organotypic slice culture methods for use in the present invention (for instance, see Alsafadi et al, 2020, Am J Respir Cell Mol Biol 62, 681-691; Liu et al, 2019, Respir Res 20, 162; WO2012/093173; Switalla et al, Toxicology in vitro, 27, 2013, 798-803; Lauenstein et al, Toxicology in vitro, 28, 2014, 588-599).

The organotypic slice can be prepared from a biopsy or tissue resection from a subject, especially a patient having a cancer. The biopsy or tissue resection can be from any organ of the subject, for instance from lung, brain, gut or skin. In a particular aspect, the organotypic slice can be prepared from a tissue resection from a subject, especially a patient having a cancer. It is particularly relevant when the patient has been treated by tumor resection. Optionally, the organotypic slice is an organotypic slice from any organ, for instance from lung, brain, gut or skin.

As used herein, the terms "subject", "individual" or "patient" are interchangeable and refer to an animal, preferably to a mammal, even more preferably to a human, including adult and child. However, the term "subject" can also refer to non-human animals, in particular mammals such as dogs, cats, horses, cows, pigs, sheeps and non-human primates, among others.

An organotypic lung slice culture refers to sections or explants of lung tissue and can employ sections of whole lung tissue or explants obtained from specific regions of the lung. Any region can be used to generate an organotypic lung slice culture. However, a preferred source of the organotypic lung slice culture is explants obtained from lobectomy.

An organotypic brain slice culture refers to sections or explants of brain tissue and can employ sections of whole brain tissue or explants obtained from specific regions of the brain. Any region can be used to generate an organotypic brain slice culture. However, a preferred source of the organotypic brain slice culture is explants obtained from specific regions of the brain, preferably the hippocampus or cortex region.

In one aspect, a set of organotypic slices can be prepared from several subjects. Alternatively, the organotypic slice(s) come(s) from one subject, especially the patient to be treated by radiotherapy.

The organotypic slices or organotypic slice cultures according to the invention may be used immediately or may be maintained in culture over multiple days using any protocol for culturing organotypic slices known in the art.

In a preferred aspect, the thickness of the organotypic slice is from 100 µm to 500 µm, preferably from 200 µm to 400 µm, more preferably from 250 µm to 350 µm, even more preferably of about 300 µm. In another preferred embodiment, the organotypic slice is prepared from a biopsy or tissue resection from a subject, for example from lung, brain, gut or skin of subject. In another preferred embodiment, the organotypic slice is a lung organotypic slice. Such a lung organotypic slice may for example be prepared from a lobectomy of a subject having a lung cancer.

In a particular aspect, the organotypic slice is a lung organotypic slice cultured in a precision-cut lung slice (PCLS) medium comprising DMEM F12 (31331-028) supplemented with 1% SVF (CVFSVF00-0U), 1% penicillin/streptomycin (CABPES01-0U), 1% non-essential amino acids (11140-035) and 1% L-glutamine (25030-024), and cultured at 37°C in 5% CO2 up to 72 h.

The step of irradiating an organotypic slice according to the invention, may be performed using any radiation therapy as described herein. In a particular aspect, the organotypic slices are submitted to irradiation no more than 3 or 2 days after tissue sampling, preferably no more than 1 day, 20, 18, 16, 14, 12, 10, 8, 6, 4 or 2 hours. Typically, the irradiation step is performed using FLASH radiotherapy (FLASH-RT), FLASH proton irradiation, proton radiotherapy, proton minibeam radiation therapy, gamma rays, brachytherapy, unsealed source radiotherapy, tomotherapy or external beam radiotherapy selected from superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT) or 3-dimensional conformal radiation therapy (3D-CRT). In a preferred embodiment, the step of irradiating an organotypic slice is performed using FLASH radiotherapy (FLASH-RT).

More generally, the radiation dose used by the invention is preferably from 3 to 20 Gy, from 3 to 18 Gy, more preferably from 3 to 10 Gy, and even more preferably from 3 to 9 Gy.

In another aspect, the irradiation time is from 8 to 2000 ms.

In another aspect, the dose of radiation is preferably from 3 to 20 Gy, from 3 to 18 Gy, more preferably from 3 to 10 Gy, and even more preferably from 3 to 9 Gy and the irradiation time is from 8 to 2000 ms.

In a particular aspect, when the irradiation step is performed by FLASH radiotherapy, the invention preferably uses 1, 2 or 3 pulses of radiation, and the dose of radiation is preferably from 3 to 6 Gy per pulse, preferably of about 3 Gy/pulse.

In another particular aspect, when the irradiation step is performed by FLASH radiotherapy, the invention uses a pulse repetition frequency from 1 to 250 Hz, preferably from 10 to 100 Hz, more preferably of about 100Hz.

In a further particular aspect, when the irradiation step is performed by FLASH radiotherapy, the invention preferably uses 1, 2 or 3 pulses of radiation, the dose of radiation is preferably from 3 to 6 Gy per pulse, preferably of about 3 Gy/pulse, and a pulse repetition frequency is from 1 to 250 Hz, preferably from 10 to 100 Hz, preferably of about 100Hz.

### Cell proliferation measure

The methods and uses for assessing the radiation-induced toxicity according to the present invention, are based on a measure of cell proliferation in the irradiated organotypic slice.

The step of measuring cell proliferation may be performed by using any technique of quantitative or qualitative analysis of cell proliferation, known in the art. In particular, the invention may use any cell proliferation assay such as any labeling, immunolabeling, radiolabeling, histological, or immunohistological assay or a combination thereof. In particular embodiments, the following cell proliferation assays can be used for measuring cell proliferation in organotypic slices according to the invention: DNA synthesis assay (detecting DNA synthesis by measuring incorporation in DNA of non-radioactive molecules, e.g., Ethynyl-2'-deoxyuridine (EdU), 5-bromo-2'-deoxyuridine (BrdUrd), etc., or radioactive molecules, e.g., tritiated thymidine ([3H]TdR), etc.); a cell proliferation marker assay (for example using an antibody against a nuclear protein associated with cellular proliferation, such as KI-67, PCNA, topoisomerase IIB, phosphorylated histone H3, etc.); a metabolic activity assay (for example measuring the activity of lactate dehydrogenase that increases during cell proliferation; and/or using tetrazolium salts such as MTT, XTT, MTS or WST1, or using Alamar Blue), and/or an ATP concentration assay (e.g., using the bioluminescent luciferase and its substrate), etc.

In a preferred embodiment, the step of measuring cell proliferation is performed by counting a number of dividing cells in the irradiated organotypic slice. Counting of dividing cells may be performed using any cell proliferation assay known in the art, preferably using an assay based on incorporation of an analog of a nucleotide or a nucleoside such as an analog of thymidine, e.g., Ethynyl-2'-deoxyuridine (EdU).

In the most preferred embodiment, the invention measures cell proliferation by using an assay based on incorporation of a 5-ethynyl-2'-deoxyuridine (EdU).

The step of measuring cell proliferation in the irradiated organotypic slice according to the invention, may comprise measuring a number, a quantity, a relative amount, a proportion, a percentage or a nature of proliferating or dividing cells. The measuring of cell proliferation may also comprise measuring the intensity of dividing cell labeling, verifying the morphology of dividing cells and/or verifying dividing cell density, using any microscopy technique known in the art.

In specific embodiments, the measuring cell proliferation is performed from 18 to 30 hours after irradiation, preferably from 20 to 28 hours after irradiation, still more preferably from 22 to 26 hours after irradiation, even more preferably about 24 hours after irradiation.

Optionally, the measure of the cell proliferation, especially by an assay based on EdU can be combined with other additional assays, for instance assays measuring the cell mortality, the apoptosis, the cell viability or other assays measuring the cell proliferation.

### Determining the radiation-induced toxicity

Within the context of the present invention, the term **"radiation-induced toxicity"** or **"radiotoxicity"** designates any toxic or adverse side effect induced by radiation which may be observed in a subject treated by radiation therapy or in an organotypic slice which was irradiated. In particular, the term radiotoxicity includes any toxic or adverse side effect of radiation, which may be observed in the irradiated organotypic slice comprising healthy tissue. The radiotoxicity also includes any toxic or adverse side effect of radiation, which may be observed in the irradiated organotypic slice comprising healthy tissue and cancer tissue.

The inventors have surprisingly discovered that radiation-induced toxicity may be assessed and determined rapidly with good reproducibility and sensitivity on the basis of cell proliferation measured in the irradiated organotypic slice, and more particularly on the basis of cell proliferation measured in the irradiated organotypic slice comprising healthy tissue.

Accordingly, the methods and uses for assessing radiation-induced toxicity according to the invention allow to determine the radiation-induced toxicity on the basis of cell proliferation measured in the irradiated organotypic slice comprising healthy tissue, said measured cell proliferation, in the irradiated organotypic slice comprising healthy tissue, being indicative of radiation-induced toxicity.

In a particular embodiment, the determining the radiation-induced toxicity according to the invention comprises measuring cell proliferation in the irradiated organotypic slice comprising healthy tissue according to any cell proliferation assay as described herein. In particular embodiments, the determining the radiation-induced toxicity according to the invention comprises measuring cell proliferation in the irradiated organotypic slice comprising healthy tissue, by measuring a number, a quantity, a relative amount, a proportion, a percentage or a nature of proliferating or dividing cells.

Preferably, the determining the radiation-induced toxicity according to the invention comprises measuring cell proliferation in the irradiated organotypic slice comprising healthy tissue, by counting a number of dividing cells in the irradiated organotypic slice, preferably using an assay based on incorporation of a 5-ethynyl-2'-deoxyuridine (EdU).

In a further embodiment, the determining the radiation-induced toxicity according to the invention is provided by measuring cell proliferation in a test irradiated organotypic slice sample comprising healthy tissue, and by comparing the cell proliferation in said test irradiated organotypic slice sample with a cell proliferation in a reference sample. For example, the determining the radiation-induced toxicity according to the invention may be provided by measuring a number, a quantity, a relative amount, a proportion, a percentage or a nature of proliferating or dividing cells in a test irradiated organotypic slice sample, as described herein, and by comparing the number, the quantity, the relative amount, the proportion, the percentage or the nature of proliferating or dividing cells in said test irradiated organotypic slice sample with a number, a quantity, a relative amount, a proportion, a percentage or a nature of proliferating or dividing cells in a reference sample.

Within the context of the present invention, the term **"reference"** or **"reference sample"** designates any sample having a reference value or control value or standard value. The reference value may be a median or average value observed in samples from reference subjects or a median or average value observed in control samples. The reference sample may be irradiated or not. Preferably, the reference sample is an organotypic slice sample which may comprise healthy tissue and/or cancer tissue. The reference sample may be from a subject treated or not treated by radiotherapy. The reference sample may be from the same subject as the test sample, or from a different subject. For example, the reference sample may be from the same subject, obtained at an earlier stage of radiation therapy that the test sample, or before the treatment by radiation therapy; or a sample from another subject treated with a same or different dose of radiation. The step of comparing cell proliferation in the test and the reference samples, may further comprise monitoring cell proliferation in both samples and detecting a change in cell proliferation. The change in cell proliferation may be a change in the number, the quantity, the relative amount, the proportion, the percentage or the nature of proliferating or dividing cells. The term "change" in cell proliferation may comprise a decrease or increase of cell proliferation which may for example correspond to the decreased or increased number, quantity, relative amount, proportion, or percentage of dividing cells.

Further measurements of cell proliferation can be compared to the previous measurements of cell proliferation in a sample from the same or different subject, or to the standard measurements in order to monitor cell proliferation.

Optionally, the reference sample can be representative of a sample presenting a radiation-induced toxicity and/or can be representative of a sample which does not present a radiation-induced toxicity. Optionally, several reference samples can be used and present various radiation-induced toxicity, for instance an increasing radiation-induced toxicity among said reference samples or a decreasing radiation-induced toxicity among said reference samples. Then, by comparing the cell proliferation of the tested organotypic slice to the one of one or several reference samples, it is possible to determine that radiation-induced toxicity in the subject from whom the organotypic slice has been prepared.

The radiation-induced toxicity is determined as "high" when the cell proliferation measured in the test irradiated organotypic slice sample comprising healthy tissue, indicates a low number, quantity, relative amount, proportion, or percentage of dividing cells in comparison to the reference sample; and/or the radiation-induced toxicity is determined as "low" when the cell proliferation measured in the test irradiated organotypic slice sample comprising healthy tissue, indicates a high or similar number, quantity, relative amount, proportion, or percentage of dividing cells in comparison to the reference sample. Thus, in a preferred embodiment, a decrease in the number of dividing cells allows to determine a high radiotoxicity, and an increase or stable level in the number of dividing cells allows to determine a low radiotoxicity.

In a further specific embodiment, the invention relates to a method for assessing radiation-induced toxicity, comprising the following steps:
(i) providing an organotypic slice sample comprising healthy tissue from a subject,
(ii) culturing the organotypic slice sample in an appropriate culture medium,
(iii) irradiating the organotypic slice culture, preferably using FLASH radiotherapy,
(iv) monitoring cell proliferation in the sample from 18 to 30 hours after the step (iii), preferably from 20 to 28 hours after the step (iii), still more preferably from 22 to 26 hours after the step (iii), even more preferably about 24 hours after the step (iii), and
(v) comparing the cell proliferation profile of the sample to a predetermined cell proliferation profile, wherein the radiation-induced toxicity is assessed based on the comparison between the cell proliferation profile of the irradiated organotypic slice culture and the predetermined profile.

In a further specific embodiment, the present invention relates to a method for assessing radiation-induced toxicity by using a lung organotypic slice assay developed by the inventors. Such a lung assay relies on organotypic lung slices obtained from mice. Lung slices are treated with different radiations modalities and the proportion of dividing cells is analyzed 18-30 hours after treatment. Samples processing and computational analysis take another 3 to 4 days, thus allowing obtention of final quantitative results within a week. The organotypic lung slices assay developed by the inventors present at least three main advantages over existing methods (such as fibrosis development that last several months and is time consuming and poorly sensitive): (i) rapidity of the method, and (ii) the limited number of animals required that allow, in an ethical way, to screen many different conditions, (iii) the lower variability between samples as all slices in a study are generated from one single animal; and (iv) the sensitivity allowing to detect low variation of radiation-induced toxicity. The assay can also be used with subject biopsy samples or lobectomy samples or in order to evaluate individual radiosensitivity when, for example, a re-irradiation can be proposed.

The method for assessing radiation-induced toxicity according to the present invention can be used for testing, comparing and developing radiotherapy regimens and to optimize them for reducing the radiation-induced toxicity. In the goal, a set of organotypic slices is used.

The method for assessing radiation-induced toxicity according to the present invention can be used for determining the radiation-induced toxicity in a particular subject, especially a patient having a cancer. Thus, depending of the determined radiation-induced toxicity for said subject, it would be possible to establish a proper personalized medicine. For instance, is this subject suitable/adapted for radiotherapy? It is necessary to modulate the radiotherapy regimen? Which radiotherapy is the more appropriate? Which dose of radiation is the more appropriate from this subject?

As used herein, the terms "subject", "individual" or "patient" are interchangeable and refer to an animal, preferably to a mammal, even more preferably to a human, including adult and child. However, the term "subject" can also refer to non-human animals, in particular mammals such as dogs, cats, horses, cows, pigs, sheeps and non-human primates, among others. In a particular aspect, the subject has a cancer.

As used herein, the term "cancer" refers to any cancer that may affect any one of the following tissues or organs: breast; liver; kidney; heart, mediastinum, pleura; floor of mouth; lip; salivary glands; tongue; gums; oral cavity; palate; tonsil; larynx; trachea; bronchus, lung; pharynx, hypopharynx, oropharynx, nasopharynx; esophagus; digestive organs such as stomach, intrahepatic bile ducts, biliary tract, pancreas, small intestine, colon; rectum; urinary organs such as bladder, gallbladder, ureter; rectosigmoid junction; anus, anal canal; skin; bone; joints, articular cartilage of limbs; eye and adnexa; brain; peripheral nerves, autonomic nervous system; spinal cord, cranial nerves, meninges; and various parts of the central nervous system; connective, subcutaneous and other soft tissues; retroperitoneum, peritoneum; adrenal gland; thyroid gland; endocrine glands and related structures; female genital organs such as ovary, uterus, cervix uteri; corpus uteri, vagina, vulva; male genital organs such as penis, testis and prostate gland; hematopoietic and reticuloendothelial systems; blood; lymph nodes; thymus.

The term "cancer" according to the invention comprises leukemias, seminomas, melanomas, teratomas, lymphomas, non-Hodgkin lymphoma, neuroblastomas, gliomas, adenocarninoma, mesothelioma (including pleural mesothelioma, peritoneal mesothelioma, pericardial mesothelioma and end stage mesothelioma), rectal cancer, endometrial cancer, thyroid cancer (including papillary thyroid carcinoma, follicular thyroid carcinoma, medullary thyroid carcinoma, undifferentiated thyroid cancer, multiple endocrine neoplasia type 2A, multiple endocrine neoplasia type 2B, familial medullary thyroid cancer, pheochromocytoma and paraganglioma), skin cancer (including malignant melanoma, basal cell carcinoma, squamous cell carcinoma, Kaposi's sarcoma, keratoacanthoma, moles, dysplastic nevi, lipoma, angioma and dermatofibroma), nervous system cancer, brain cancer (including astrocytoma, medulloblastoma, glioma, lower grade glioma, ependymoma, germinoma (pinealoma), glioblastoma multiform, oligodendroglioma, schwannoma, retinoblastoma, congenital tumors, spinal cord neurofibroma, glioma or sarcoma), skull cancer (including osteoma, hemangioma, granuloma, xanthoma or osteitis deformans), meninges cancer (including meningioma, meningiosarcoma or gliomatosis), head and neck cancer (including head and neck squamous cell carcinoma and oral cancer (such as, e.g., buccal cavity cancer, lip cancer, tongue cancer, mouth cancer or pharynx cancer)), lymph node cancer, gastrointestinal cancer, liver cancer (including hepatoma, hepatocellular carcinoma, cholangiocarcinoma, hepatoblastoma, angiosarcoma, hepatocellular adenoma and hemangioma), colon cancer, stomach or gastric cancer, esophageal cancer (including squamous cell carcinoma, larynx, adenocarcinoma, leiomyosarcoma or lymphoma), colorectal cancer, intestinal cancer, small bowel or small intestines cancer (such as, e.g., adenocarcinoma lymphoma, carcinoid tumors, Kaposi's sarcoma, leiomyoma, hemangioma, lipoma, neurofibroma or fibroma), large bowel or large intestines cancer (such as, e.g., adenocarcinoma, tubular adenoma, villous adenoma, hamartoma or leiomyoma), pancreatic cancer (including ductal adenocarcinoma, insulinoma, glucagonoma, gastrinoma, carcinoid tumors or vipoma), ear, nose and throat (ENT) cancer, breast cancer (including HER2-enriched breast cancer, luminal A breast cancer, luminal B breast cancer and triple negative breast cancer), cancer of the uterus (including endometrial cancer such as endometrial carcinomas, endometrial stromal sarcomas and malignant mixed Müllerian tumors, uterine sarcomas, leiomyosarcomas and gestational trophoblastic disease), ovarian cancer (including dysgerminoma, granulosa-theca cell tumors and Sertoli-Leydig cell tumors), cervical cancer, vaginal cancer (including squamous-cell vaginal carcinoma, vaginal adenocarcinoma, clear cell vaginal adenocarcinoma, vaginal germ cell tumors, vaginal sarcoma botryoides and vaginal melanoma), vulvar cancer (including squamous cell vulvar carcinoma, verrucous vulvar carcinoma, vulvar melanoma, basal cell vulvar carcinoma, Bartholin gland carcinoma, vulvar adenocarcinoma and erythroplasia of Queyrat), genitourinary tract cancer, kidney cancer (including clear renal cell carcinoma, chromophobe renal cell carcinoma, papillary renal cell carcinoma, adenocarcinoma, Wilm's tumor, nephroblastoma, lymphoma or leukemia), adrenal cancer, bladder cancer, urethra cancer (such as, e.g., squamous cell carcinoma, transitional cell carcinoma or adenocarcinoma), prostate cancer (such as, e.g., adenocarcinoma or sarcoma) and testis cancer (such as, e.g., seminoma, teratoma, embryonal carcinoma, teratocarcinoma, choriocarcinoma, sarcoma, interstitial cell carcinoma, fibroma, fibroadenoma, adenomatoid tumors or lipoma), lung cancer (including small cell lung carcinoma (SCLC), non-small cell lung carcinoma (NSCLC) including squamous cell lung carcinoma, lung adenocarcinoma (LUAD), and large cell lung carcinoma, bronchogenic carcinoma, alveolar carcinoma, bronchiolar carcinoma, bronchial adenoma, lung sarcoma, chondromatous hamartoma and pleural mesothelioma), sarcomas (including Askin's tumor, sarcoma botryoides, chondrosarcoma, Ewing's sarcoma, malignant hemangioendothelioma, malignant schwannoma, osteosarcoma and soft tissue sarcomas), soft tissue sarcomas (including alveolar soft part sarcoma, angiosarcoma, cystosarcoma phyllodes, dermatofibrosarcoma protuberans, desmoid tumor, desmoplastic small round cell tumor, epithelioid sarcoma, extraskeletal chondrosarcoma, extraskeletal osteosarcoma, fibrosarcoma, gastrointestinal stromal tumor (GIST), hemangiopericytoma, hemangiosarcoma, Kaposi's sarcoma, leiomyosarcoma, liposarcoma, lymphangiosarcoma, lymphosarcoma, malignant peripheral nerve sheath tumor (MPNST), neurofibrosarcoma, plexiform fibrohistiocytic tumor, rhabdomyosarcoma, synovial sarcoma and undifferentiated pleomorphic sarcoma, cardiac cancer (including sarcoma such as, e.g., angiosarcoma, fibrosarcoma, rhabdomyosarcoma or liposarcoma, myxoma, rhabdomyoma, fibroma, lipoma and teratoma), bone cancer (including osteogenic sarcoma, osteosarcoma, fibrosarcoma, malignant fibrous histiocytoma, chondrosarcoma, Ewing's sarcoma, malignant lymphoma and reticulum cell sarcoma, multiple myeloma, malignant giant cell tumor chordoma, osteochronfroma, osteocartilaginous exostoses, benign chondroma, chondroblastoma, chondromyxoid fibroma, osteoid osteoma and giant cell tumors), hematologic and lymphoid cancer, blood cancer (including acute myeloid leukemia, chronic myeloid leukemia, acute lymphoblastic leukemia, chronic lymphocytic leukemia, myeloproliferative diseases, multiple myeloma and myelodysplasia syndrome), Hodgkin's disease, non-Hodgkin's lymphoma and hairy cell and lymphoid disorders, and the metastases thereof. Optionally, the cancer can be selected in the group consisting of rectal cancer, colorectal cancer, stomach cancer, head and neck cancer, thyroid cancer, cervical cancer, uterine cancer, breast cancer.

In a particular aspect, the cancer can be selected from the group consisting of lung cancer, gastrointestinal cancer, brain cancer and melanoma. More particularly, the cancer can be a lung cancer including non-small cell lung cancer (NSCLC), bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; a brain cancer including astrocytoma, medulloblastoma, glioma, ependymoma, germinomapinealoma, glioblastoma multiform, oligodendroglioma, Schwannoma, retinoblastoma, congenital tumors, and Spinal cord tumors(neurofibroma, meningioma, glioma, Sarcoma); a gastrointestinal cancer including rectal cancer, colorectal cancer and stomach cancer.

### Other methods

As mentioned before, the information obtained by performing an *ex vivo* method for assessing radiation-induced toxicity, as described herein, may be further used to aid in the determining a subject's responsiveness to radiotherapy, and to select an optimal treatment for the subject, in particular a subject as defined above.

In this regard, the present invention also provides a method for selecting a subject responsive to radiotherapy, comprising an *ex-vivo* method for assessing radiation-induced toxicity of said subject, as described herein.

In a particular embodiment, the invention relates to a method for selecting a subject as adapted for radiotherapy, the method comprising the following steps:
(a) assessing radiation-induced toxicity of said subject by performing an *ex vivo* method for assessing radiation-induced toxicity, as described herein, said method comprising;
   - irradiating an organotypic slice comprising healthy tissue;
   - measuring cell proliferation in the irradiated organotypic slice; and
   - determining the radiation-induced toxicity based on the measured cell proliferation in the irradiated organotypic slice; and
(b) selecting the subject as adapted for radiotherapy if the radiation-induced toxicity of said subject is low and/or selecting the subject being not adapted for radiotherapy if the radiation-induced toxicity of said subject is high.

In another particular embodiment, the invention relates to a method for determining a subject's responsiveness to radiotherapy, comprising the following steps:
(i) providing an organotypic slice sample, preferably an organotypic lung slice sample,
(ii) irradiation of the sample,
(iii) monitoring cell proliferation in the sample after the irradiation of step (ii),
(iv) comparing the cell proliferation profile of the sample to a predetermined cell proliferation profile, wherein the subject's responsiveness is determined based on the comparison between the profile of the cell proliferation in the irradiated organotypic slice sample and the predetermined profile.

Optionally, these methods can comprise an additional step of applying radiotherapy to the selected patient.

The above methods may further comprise a step of adapting the radiation therapy in the subject responsive to radiotherapy and/or defining a personalized or optimized radiation therapy regimen for the subject and/or treating the subject using the personalized or optimized radiation therapy and/or determining the efficacy of the personalized or optimized radiation therapy. The personalized or optimized radiation therapy may further be combined with another anti-cancer treatment.

As mentioned above, the present invention also provides methods for aiding in the prognosis, diagnosis and/or treatment of cancer in a subject responding to radiotherapy, as well as for defining personalized/optimal treatment regimen of cancer in a subject.

Depending on the level of radiotoxicity for a subject, the radiotherapy procedure can be optimized for the subject, individually.

In a particular embodiment, the method for assessing radiation-induced toxicity according to the invention is suitable for use in a method for providing information in order to adapt radiotherapy in a subject. Such information may concern the most appropriate radiation type, delivery means and/or the radiation dose. The method for providing information for adapting radiotherapy in a subject according to the invention, may thus comprise a step of performing the method for assessing radiation-induced toxicity on an organotypic slice from said subject, as detailed herein, and defining a personalized or optimized radiation therapy treatment for the subject based on radiation-induced toxicity of said subject.

For instance, if the patient has no radiation-induced toxicity or a weak one, then this patient can be treated by radiotherapy. On the contrary, if the patient presents a radiation-induced toxicity, especially a significant or high radiation-induced toxicity, thus this patient can be excluded from a further radiotherapy or the radiotherapy can be adapted to reduce radiation-induced toxicity, for instance by selecting particular radiotherapy and by lowering the dose of irradiation.

The methods of the present invention are of particular interest in cancer for which a first treatment includes a tumor resection and for which, in case of cancer recurrence or relapse, a radiotherapy can be considered as a treatment.

In a particular embodiment, the invention relates to a method for providing information for adapting radiotherapy in a subject based on radiation-induced toxicity of said subject, the method comprising the following steps:
(a) assessing radiation-induced toxicity of said subject by performing an ex vivo method for assessing radiation-induced toxicity, as described herein, said method comprising;
   - irradiating an organotypic slice comprising healthy tissue;
   - measuring cell proliferation in the irradiated organotypic slice; and
   - determining the radiation-induced toxicity based on the measured cell proliferation in the irradiated organotypic slice; and
(b) defining a personalized or optimized radiation therapy regimen for the subject based on radiation-induced toxicity of said subject.

In another particular embodiment, the invention relates to a method for treating a subject having a cancer by radiotherapy, the method comprising the following steps:
(a) assessing radiation-induced toxicity of said subject by performing an ex vivo method for assessing radiation-induced toxicity, as described herein, said method comprising;
   - irradiating an organotypic slice comprising healthy tissue;
   - measuring cell proliferation in the irradiated organotypic slice; and
   - determining the radiation-induced toxicity based on the measured cell proliferation in the irradiated organotypic slice; and
(b) treating said subject by radiation therapy, optionally with a personalized or optimized radiation therapy regimen based on radiation-induced toxicity of said subject, preferably the subject having a cancer relapse.

In a specific embodiment, the method according to the invention is used to prevent cancer relapse. Indeed, in case of cancer relapse, all the information about the previous responsiveness of the subject to radiation therapy may be available before cancer relapse, and thus the protocol of the future radiation therapy (including all the parameters of radiation therapy, e.g., dose, duration time and/or type of radiation), may be accordingly adapted.

In other particular embodiment, the above methods according to the invention may further comprise selecting the dose of radiation or the type of radiation which is the most suitable for the subject. In a preferred embodiment, the selected radiation dose is administered by FLASH irradiation.

In an additional aspect, the method of the present invention can be used for determining if the cancer/tumor is responsive to radiotherapy. In this context, the organotypic slice comprises tumor tissue in addition to healthy tissue. Similarly to the previous methods, the organotypic slice is irradiated and the cell proliferation is measured. Thus, based on the cell proliferation in the organotypic slice comprising tumor tissue, it can be determined if the tumor/cancer is responsive to radiotherapy.

The advantage is that the method allows to obtain simultaneously the radiation-induced toxicity on a healthy tissue and the radiation-induced toxicity on a tumor tissue. Thus, based on these information, it is possible 1) to test and compare radiotherapy regimens and to optimize them for reducing the radiation-induced toxicity on healthy tissue while optimizing the radiation-induced toxicity on tumor tissue; 2) to define a personalized or optimized radiation therapy regimen for a subject having a cancer based on radiation-induced toxicity of said subject in healthy tissue and in tumor tissue from said subject.

Further aspects and advantages of the invention will be disclosed in the following experimental section, which illustrates the invention and does not limit the scope of the present application.

### EXAMPLES

### MATERIELS AND METHODS

**Mice and ethics statement.** Studies were performed in accordance with the European Community recommendations (2010/63/EU) for the care and use of laboratory animals. The experimental procedures were specifically approved by the Ethics Committee of the Institut Curie CEEA-IC #118 (authorization number APAFiS#32674-2021080916494690 given by the National Authority) in compliance with international guidelines. Females C57BL/6J mice purchased from Charles River Laboratories at 6 weeks of age were housed in the Institut Curie animal facilities.

**Precision-Cut Lung Slices obtention and culture.** Precision-cut lung slices (PCLS) are generated as described previously in the literature (Alsafadi et al. Am J Respir Cell Mol Biol. 2020 Jun;62(6):681-691). Adult female C57BL/6J mice were anesthetized by intraperitoneal injection of a ketamine/xylazine. Blood is then flushed through intracardiac injection of Phosphate buffered saline (PBS) and the trachea is exposed to inject 2 ml of 2.5 % low-melting agarose (A9414-50G) diluted in PCLS medium (DMEM F12 (31331-028) supplemented with 1% SVF (CVFSVF00-0U), 1% penicillin/streptomycin (CABPES01-0U), 1% non-essential amino acids (11140-035) and 1% L-glutamine (25030-024)). Once the agarose is solidified, lungs are removed from the chest cavity and 6 to 8 mm punches are made from each lobe individually. Tissue punches are embedded in 5% agarose and 300 µm slices are made with a vibratome (Leica VT1000S) with adjusted parameters (90 Hz for the cutting frequency and 2.25 mm/s) for the cutting speed) as previously described (Alsafadi et al. Am J Respir Cell Mol Biol. 2020 Jun;62(6):681-691). Slices are incubated 30 min at 37°C before changing the medium to remove agarose excess. Around 50 slices are obtained from the whole lung and placed into a 24-well plate containing 500 µL of PCLS medium ((DMEM F12 (31331-028) supplemented with 1% SVF (CVFSVF00-0U), 1% penicillin/streptomycin (CABPES01-0U), 1% non-essential amino acids (11140-035) and 1% L-glutamine (25030-024)) and cultured at 37°C in 5% CO2 up to 72 h.

**PCLS irradiation.** PCLS were irradiated in 24-well culture plates using the electron linear accelerator ElectronFLASH 4000 built by Sordina IORT company, capable of conventional and ultra-high dose-rate (FLASH) irradiation. The energy of the electron beam was set at 7 MeV. In conventional dose-rate modality, the mean dose-rate was around 25 Gy.min-1, with a Pulse Repetition Frequency of 10 Hz. In FLASH modality, the mean dose-rate was above 400 Gy.s-1, with a Pulse Repetition Frequency of 100 Hz. The dose delivered to the PCLS in the 24-well plates was determined using EBT-XD Gafchromic films placed at the bottom the wells. In the beam-axis direction, a build-up thickness of 6 mm of PMMA was added under the plate, in order to position the culture plates at the plateau part of the Depth Dose Distribution, and hence, the dose inside the wells was equal to the dose read on a film placed under the plate. For the following PCLS irradiations, films under the plate served as a cross-check on the dose delivered to the PCLS over time. In the lateral direction, the doses measured in each well exhibit a standard deviation < 3% (k=1). The average dose over all the wells was used as prescribed dose. In FLASH modality, this determined the dose-per-pulse, which was estimated at 3 Gy.pulse-1 in this set-up. The total dose delivered to the PCLS is planned by choosing the number of pulses. In conventional modality, this dose was correlated units of electrical charges counted by a pair of monitor chambers installed at the exit window of the accelerator. The beam was automatically stopped when the monitor units reached the prescribed value.

**Cell division assay.** To estimate the proportion of cells that replicate after irradiation, we used a Click-IT chemistry protocol to monitor cell proliferation using 5-ethynyl-2'-deoxyuridine (EdU) incorporation (BCK-EdUPro-FC647). EdU is a thymidine analogue that will be incorporated into DNA during replication. Irradiated PCLS are incubated in 500 µL of culture medium containing 10 µM EdU. After the desired incubation time (i.e. 24, 48 or 72 hours), PCLS are washed twice in PBS, fixed in 4% paraformaldehyde (PFA) for 1 hour and washed in PBS containing 3% bovine serum albumin (BSA). To reveal EdU incorporation, PCLS are permeabilized for 1 hour with a saponin buffer and stained with the Click-IT cocktail containing the azide coupled to a fluorophore. Then, PCLS are washed in PBS and incubated with a nuclear dye (e.g. DAPI). For imaging, PCLS are transferred into a glass support adapted for microscopy (µ-Slide 4 Well Glass Bottom, Ibidi) and imaged on an inverted Nikon Spinning disk TIRF-FRAP using a 10X objective. Per PCLS, we acquired 3 to 5 fields of view, each containing 50 stacks spaced by 3 µm. To minimize signal variability, samples from a single experiment are acquired the same day. To quantify the proportion of EdU positive cells, we applied a semi-automatic method combining 3D reconstruction and segmentation of the nuclei using IMARIS software (Bitplane). Briefly, threshold for DAPI and EdU staining was defined on the control PCLS images to segment both signals and applied to all the images of the same experiment. Then, we used the spot detection function to count the number of EdU+ nuclei with a size over 11 µm per FOV. Similar regions of the lung parenchyma were analyzed across different conditions and, after checking the number of nuclei per FOV did not in change drastically, we directly compared the proportion of EdU+ cells between conditions.

**Primary human lung basal stem cells (LBSC) isolation.** Bronchial rings used for the isolation of the human primary lung basal stem cell were provided by hospital Institut Mutualiste Montsouris (Paris) from freshly tissue resected during lobectomies of subjects who underwent surgery for lung cancer. Collection, storage and use of tissues were performed in agreement with the declared protocol under the reference EUdract 2017-A03081-52 and was approved by the Ethics Committee CPP SUD-EST I. Subjects provided written informed consent to permit the use of the material for research. LBSC were grown in BEBM medium (Lonza^{®}) supplemented with Bovine Pitituary Extract, Hydrocortisone, human Epidermal Growth Factor, Epinephrine, Transferrin, Insulin, Retinoic Acid, Triiodothyronine, and Gentamicin/Amphotericin-B and maintained at 37°C in 5% CO2.

**LBSC analysis.** Cells were seeded on coverslips in 12-well plate and irradiated with electron linear accelerator ElectronFLASH 4000 at the determined doses. One day after irradiation, EdU (10 µM) was adding in the culture medium. After the desired incubation time, coverslips were washed in PBS and fixed in 4% paraformaldehyde (PFA). As for the PCLS, EdU incorporation was revealed with the BCK-EdUPro-IM647 kit following the permeabilization and staining steps, adding a DAPI costaining. Coverslips were mounted on a microscopy slide and were imaged on a Zeiss Axio Imager 2 microscope using the 10X objective. For each condition, 12 fields were acquired and analyzed with a macro developed on ImageJ to determine the number of EdU positive nuclei as a proportion of the total number of nuclei.

**Statistical analysis.** Statistical analyses were performed using the ggpubr package (https://rpkgs.datanovia.com/ggpubr/) in R. For our EdU+ nuclei count data as well as for the estimation of the viability of organotypic slices (ethidium nuclei/calcein volume), we assumed PCLS are dependent (organotypic slices are from the same biological sample) and our data do not follow a normal distribution. Comparisons of the means of these counts for the FLASH and CONV modalities were therefore done by a Wilcoxon test. The p-value <0.05 was considered significant. The number of biological replicates is detailed in each figure.

### RESULTS

Historical studies on lung toxicities investigated the impact of radiation on the development of pneumonia and pulmonary fibrosis (Travis, 1980, International Journal of Radiation Oncology∗Biology∗Physics 6, 345-347). These complex *in vivo* analyses are time consuming (e.g. radiation induced pulmonary fibrosis develops in 20 to 24 weeks) and require a large number of animals, a key ethical issue that will limit the feasibility of these kind of studies in the future. To circumvent these problems, the inventors used PCLS (precision-cut lung slice) from mouse lung, obtained as previously described (Alsafadi et al. Am J Respir Cell Mol Biol. 2020 Jun;62(6):681-691) to evaluate radiation toxicities. The inventors obtained around 50 slices at a thickness of 300 µm from a single mouse lung and they first analyzed the number of dividing cells for the first three days of culture.

### Example 1. Characterization of proliferation within PCLS.

The capacity of regeneration of the tissue depends upon the capacity of some stem cells to proliferate and differentiate to reconstruct damaged tissue. The proliferating cells within PCLS are detected using different exposure time to 5-ethynyl-2'-deoxyuridine (EdU) added to growth medium. EdU is a thymidine analogue that will be incorporated into DNA during replication. Quantification of the number of EdU positive cells is determined using a semi-automatic method combining 3D reconstruction and segmentation of nuclei using IMARIS software (Bitplane). The proportion of EdU⁺ cells, indicating the number of cells that replicate inside PCLS drastically increase after 24-48 hours in culture (Figure 1). Indeed, the number of dividing cells per field of view (FOV) shifted from 36 cells after 24 hours to 480 cells after 48 hours and 818 cells after 72 hours **(****Figure 1****).** This preliminary characterization of cell division of lung PCLS showed that, in order to limit the drifts inherent to the PCLS organotypic model, it is important to wisely choose the time of analysis to investigate radiation toxicity. To get as close as possible to physiological conditions, the inventors assume that 24 hours is the optimal timepoint to analyze the impact of radiation in this model.

### Example 2. Effect of radiation on cell division in Precision Cut Lung Slices (PCLS)

Immediately after treatment, radiation induces DNA damages and cell cycle arrest to allow irradiated cells to repair. As previously shown *in vivo* in the gut (Metcalfe et al. (2014). Cell Stem Cell 14, 149-159; Levy et al. (2020). Scientific Reports 10, 21600), the inventors hypothesize that radiation will impact the capacity of lung cells to divide during the first 24 hours in PCLS. Freshly prepared PCLS were exposed to various doses of radiation (i.e. from 0 Gy to 20 Gy). EdU was added to culture medium immediately after irradiation and incubated for 24 hours. Then, PCLS were imaged and the number of EdU⁺ cells in PCLS was assessed per condition (n = 4 PCLS with a minimum of 4 FOV each) **(****Figure 2****).** The inventors' analysis showed that, the number of EdU+ per field of view progressively decreases when radiation doses increase. At the dose of 12 Gy, less than 2 nuclei per FOV were detected and, no EdU+ cell was found after 20 Gy irradiation. Altogether, the quantitative analysis of cell division indicates that radiation impacts cell division in a dose-dependent manner in organotypic lung slices.

### Example 3. Estimation of the reproducibility of the method of assessing the toxicity of radiation therapy according to the invention.

To determine the robustness of the method according to the invention, the inventors estimated the variability between technical replica (i.e. PCLS obtained from the same mice) and between biological replica (i.e. PCLS obtained from different mice for the same condition). They thus, assessed the reproducibility of the test of the invention after a 9 Gy irradiation and determined i) the intra-PCLS variability by comparing four different PCLS obtained from the same mouse and ii) the inter-mouse variability by analyzing PCLS obtained from three different mice (Figure 3). The results indicate that the test according to the invention is highly reproducible with no significant differences detected either between PCLS obtained from the same mouse or between mice in different experiments.

### Example 4. Evaluation of the sensitivity of the method of assessing the toxicity of radiation therapy according to the invention.

To estimate the sensitivity of the method according to the invention, the inventors compared the results obtained from two different radiation modalities: conventional (CONV) and FLASH irradiation. Compared to conventional radiation, FLASH is known to spare healthy tissue, particularly the lung, while maintaining the antitumoral efficacy (Favaudon et al. 2014, Science Translational Medicine 6, 245ra93-245ra93). This FLASH effect has been observed *in vivo* and most of *in vitro* assays are not sensitive enough to detect the FLASH effect. The inventors thus analyzed, with the method of the invention, the impact of a dose of 9 Gy delivered either in CONV or FLASH conditions and compared with the results obtained with *in vitro* analysis of the primary human lung basal stem cells (LBSC) proliferation **(****Figure 4****).** The analysis with the method of the invention showed that there is a significant higher number of EdU⁺ cells 24 hours after FLASH irradiation compared to CONV and this difference was reproducibly observed in the three different mice. However, the analysis of the LBSC isolated from three different subjects did not allow to detect a difference in the proportion of EdU+ cells, suggesting that the LBSC model was not sensitive enough to highlight the FLASH effect. In summary, compared to other *in vitro* models such as the use of LBSC derived from subjects, the method of the invention is sensitive enough to detect even small difference in radiation toxicity like the one observed after FLASH radiotherapy.

### Example 5. Tested radiotoxicity analysis methods which did not prove to be relevant

### Analysis of cell viability based on metabolic activity and analysis of apoptotic death

Some methods developed by the inventors with this model did not prove to be relevant. Indeed, viability tests based on metabolic activity (PrestoHS blue assay) are adapted to cell suspensions. In the present model, this type of analysis did not reveal an increase in radiation-induced lethality under the tested conditions. In parallel to the analysis methods aiming at estimating the global mortality in lung slices after irradiation, the inventors tried to set up a follow-up of apoptotic death. However, the two strategies used (immunohistochemical detection for caspase 3 activation or specific Annexin V-Caspase 3 kit) could not be validated. Indeed, difficulties of permeabilization, diffusion of antibodies and markers, as well as the importance of background noise did not allow us to differentiate organotypic lung slices irradiated or not during the inventors' tests. Subsequently, one of our first approaches was to evaluate the viability of our organotypic slices by performing a double Ethidium/Hoechst labeling. This method aimed at estimating the ratio of dead cells (ethidium permeable) to total cells (Hoechst labeled) over the post-irradiation time for a wide range of doses. A macro analysis on ImageJ was developed to quantify this ratio from the acquired images. However, this method did not prove to be relevant. Indeed, the ratios, representing the proportion of dead cells, were very high (about 60 to 80%) for all the tested conditions including the unirradiated slices. The inventors were unable to show a relationship between the irradiation dose received and the cell death ratio with this method. A plausible hypothesis is a lack of deep Hoechst diffusion that could lead to an underestimation of the total live cell area.

### Analysis of cell viability in lung PCLS after irradiation

Finally, the inventors have implemented a method used in literature to estimate viability in organotypic lung slices based on a double labelling Ethidium/Calcein. To investigate the impact of different radiation doses on cell viability in lung organotypic slices, the inventors stained PCLS with Calcein/Ethidium after exposure to doses ranging from 3 Gy to 20 Gy. The cell viability analysis were performed 24 hours after irradiation to limit cell death induced by culture conditions. Quantitative image analysis allowed to estimate irradiation toxicity by the ratio of the number of dead cells (i.e Ethidium positive cells) on the volume of live tissue (i.e. Calcein staining). The inventors observed a dose-dependent increase in radiation-induced cell death at 24 hours after irradiation (Figure 5A-B). Interestingly, up to the dose of 6 Gy, they observed an increase in the number of ethidium positive cells per field of view with a slight decrease in the volume of Calcein. Over doses of 9 Gy, the increase in Ethidium-stained nuclei is accompanied by a decrease in the Calcein staining. However, when they wished to compare CONV and FLASH irradiation, this test was not sensitive enough to show a difference in radiotoxicity (Figure 5A-B).

## Claims

1. An *ex vivo* method for assessing radiation-induced toxicity, wherein the method comprises:
- irradiating an organotypic slice comprising healthy tissue;
- measuring cell proliferation in the irradiated organotypic slice; and
- determining the radiation-induced toxicity based on the measured cell proliferation in the irradiated organotypic slice.

2. Use of an organotypic slice comprising healthy tissue for assessing radiation-induced toxicity by measuring cell proliferation in the organotypic slice after irradiation of said organotypic slice.

3. The method of claim 1 or the use of claim 2, wherein the organotypic slice is prepared from a biopsy or tissue resection from a subject.

4. The method or use of claim 3, wherein the organotypic slice is prepared from a biopsy or tissue resection from lung, brain, gut or skin.

5. The method or use of claim 3, wherein the organotypic slice is a lung organotypic slice prepared from a lobectomy of a subject having a lung cancer.

6. The method of any one of claims 1 and 3-5 or the use of any one of claim 2-5, wherein the irradiation is performed using FLASH radiotherapy (FLASH-RT), FLASH proton irradiation, proton radiotherapy, proton minibeam radiation therapy, gamma rays, brachytherapy, unsealed source radiotherapy, tomotherapy or external beam radiotherapy selected from superficial X-rays therapy, orthovoltage X-rays therapy, megavoltage X-rays therapy, radiosurgery, stereotactic radiation therapy, Fractionated stereotactic radiation therapy, cobalt therapy, electron therapy, fast neutron therapy, neutron-capture therapy, proton therapy, intensity modulated radiation therapy (IMRT) or 3-dimensional conformal radiation therapy (3D-CRT).

7. The method or use of claim 6, wherein the irradiation is performed using FLASH radiotherapy (FLASH-RT).

8. The method of any one of claims 1 and 3-7 or the use of any one of claims 2-7, wherein the measuring cell proliferation is performed by counting a number of dividing cells in the organotypic slice.

9. The method or use of claim 8, wherein the measuring cell proliferation is performed using an assay based on incorporation of a 5-ethynyl-2'-deoxyuridine (EdU).

10. The method of any one of claims 1 and 3-9 or the use of any one of claims 2-9, wherein the measuring cell proliferation is performed from 18 to 30 hours after irradiation, preferably from 20 to 28 hours after irradiation, still more preferably from 22 to 26 hours after irradiation, even more preferably about 24 hours after irradiation.

11. The method of any one of claims 1 and 3-10 or the use of any one of claims 2-10, wherein the thickness of the organotypic slice is from 100 µm to 500 µm, preferably of about 300 µm.

12. The method of any one of claims 1 and 3-11 or the use of any one of claims 2-11, wherein the dose of radiation is from 3 to 20 Gy, preferably from 3 to 10 Gy, more preferably from 3 to 9 Gy; and/or the irradiation time is from 8 to 2000 ms.

13. A method for selecting a subject as adapted for radiotherapy, the method comprising assessing radiation-induced toxicity of said subject by performing the method according to any one of claims 1 and 3-12 on an organotypic slice from said subject, and selecting the subject as adapted for radiotherapy if the radiation-induced toxicity of said subject is low and/or selecting the subject being not adapted for radiotherapy if the radiation-induced toxicity of said subject is high.

14. A method for providing information for adapting radiotherapy in a subject based on radiation-induced toxicity of said subject, the method comprising assessing the radiation-induced toxicity of said subject by performing the method according to any one of claims 1 and 3-12 on an organotypic slice from said subject, and defining a personalized or optimized radiation therapy regimen for the subject based on radiation-induced toxicity of said subject.

15. A method for treating a subject having a cancer by radiotherapy, the method comprising assessing the radiation-induced toxicity of said subject by performing the method according to any one of claims 1 and 3-12 on an organotypic slice from said subject, and treating said subject by radiation therapy, optionally with a personalized or optimized radiation therapy regimen based on radiation-induced toxicity of said subject, preferably the subject having a cancer relapse.
